# EUROPEAN PATENT SPECIFICATION

(11) **EP 1 494 524 B1**
(45) Date of publication and mention of the grant of the patent: **29.12.2010**
(21) Application number: 03703960.9
(22) Date of filing: 23.01.2003
(51) Int. Cl.: A61M 25/01, A61D 19/02, A01K 29/00

(54) **APPARATUS FOR CREATING A PATHWAY IN AN ANIMAL**
GERÄT ZUR SCHAFFUNG EINES WEGS IN EINEM TIER
APPAREIL PERMETTANT DE CREER UNE VOIE CHEZ UN ANIMAL

(30) Priority: 03.04.2002 US 369941 P; 31.05.2002 US 161575; 14.11.2002 US 295008; 26.11.2002 US 304524
(43) Date of publication of application: 12.01.2005
(73) Proprietor: Pathway Technologies, LLC, Reno, NV 89501 (US)
(72) Inventor: ANDERSON, Donald, E., DECEASED (US); ANDERSON, Mark, E., San Ramon, CA 94583 (US); ANDERSON, Glenn, M., San Ramon, CA 94583 (US); LIM, Kang, S., Danville, CA 94506 (US)
(74) Representative: Repkow, Ines
(86) International application number: PCT/US2003/001927
(87) International publication number: WO 2003/084584

(56) References cited:
- WO-A-84/00113
- DE-A1- 2 845 202
- US-A- 3 050 060
- US-A- 3 380 453
- US-A- 4 109 659
- US-A- 4 790 814
- US-A- 5 899 848
- US-A- 6 071 231

## Description

### BACKGROUND OF THE INVENTION

The present invention relates to the field of creating a pathway into an animal. More particularly, the present invention relates to more effective methods and apparatus for safely creating pathways in mammals for applications such as artificial insemination (AI).

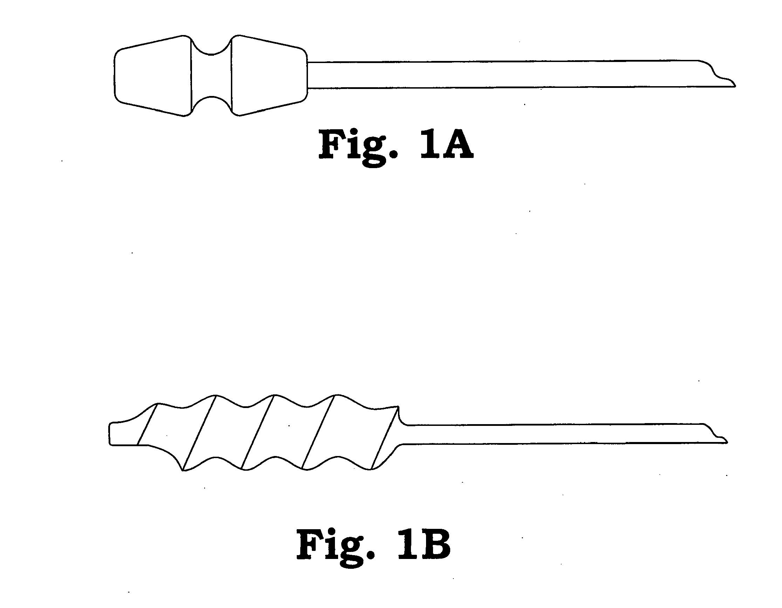

In order to feed the world population that is swelling rapidly year after year, there is an urgent need for a safer and more efficient AI of swine and other farm animals, where fresh or frozen semen and/or embryo transfer technology can be used to transfer high genetic value materials, thereby increasing the quality and quantity of the livestock litters. Figures 1A and 1B show conventional AI catheters for swine.

Unfortunately, freezing is usually necessitated by the short life span of fresh genetic materials and the logistics of distribution. Even with advanced freezing techniques, thawing causes a reduction in the mobility, motility and fertility of the spermatozoa, resulting in the need for trans-cervical intra-uterine AI to obtain commercially acceptable conception rates.

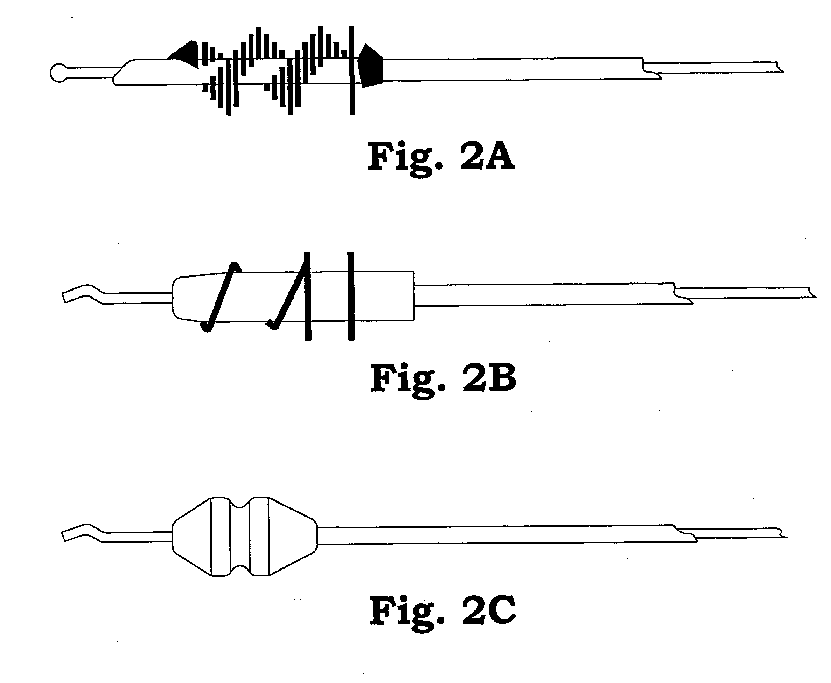

Referring to Figures 2A, 2B and 2C, a number of attempts have been made to deposit the weakened spermatozoa directly in the uterus or uterine horn by trans-cervical intra-uterine AI using rigid trans-cervical deep insemination catheters. These rigid deep insemination catheters are basically reduced diameter catheters that are enclosed and extend from within a conventional AI catheter.

The rigid deep insemination catheters are pushed and/or threaded through cervical canals using bulbous ends or slight angles on their tips in an attempt to navigate the curves and turns of the cervical canal. One inherent flaw of these rigid deep insemination catheters is their hard tips that can easily damage or puncture soft tissue areas during entry and exit procedures, often injuring or even killing the animal. Other disadvantages of these rigid catheters include the need for a professional, such as veterinarian or a highly trained technician, to perform these trans-cervical intra-uterine AI procedures, which reduces but does not substantially eliminate the risk of serious trauma and resulting sterility or death.

Hence there is a need for a safer and more effective deep trans-cervical intra-uterine AI catheter that causes minimal discomfort and risk of trauma, and does not require the services of a highly trained AI professional. Such a safer and easier-to-use AI catheter will be especially beneficial to the small farmers in third world countries who cannot afford the services of a professional.

Documents US-A-3 050 060, US-A-4 109 659, DE 26 45 202, WO 8400113, and US-A-5 899 848 disclose further prior art catheters. The catheters described in these documents are, however, either not catheters having the above mentioned desired properties and/or not catheters for opening a pathway in the cervical tract of a sow and depositing an artificial insemination fluid into the sow's body.

### SUMMARY OF THE INVENTION

To achieve the foregoing and in accordance with the present invention, an apparatus as specified in claim 1, for safer and more effective deep trans-cervical intra-uterine artificial insemination (AI) is provided. Such a deep AI catheter causes minimal discomfort and risk of trauma, and does not require the services of a highly trained AI professional

In one embodiment, a catheter is inserted into the cervical tract of the animal to begin creating a pathway in the reproductive tract of an animal. A membrane, initially positioned inside a tube section of the catheter, is extended from an opening in the tube and into the tract under pressure. The membrane extends into the tract without friction, i.e. without sliding action between the membrane and the tract, thereby reducing the discomfort and the risk of trauma or injury to the animal. When the membrane is fully extended into the tract, pressure causes the tip of the membrane to open thereby releasing the AI fluid and depositing the genetic material suspended in the fluid into the reproductive tract.

In accordance with one aspect of the invention, deployment of the membrane is facilitated by its taper. Hence in some embodiments, tapering can be accomplished by reducing the wall thickness and/or diameter of the membrane towards its tip.

In addition to AI and embryo transplant, other applications for the pathway include other therapeutic, diagnostic or procedures, such as introducing fluoroscopic cameras, instruments, and drug delivery. Note that the various features of the present invention, including the extending membrane and the nozzle, can be practiced alone or in combination. These and other features of the present invention will be described in more detail below in the detailed description of the invention and in conjunction with the following figures.

### BRIEF DESCRIPTION OF THE DRAWINGS

The present invention is illustrated by way of example, and not by way of limitation, in the figures of the accompanying drawings and in which like reference numerals refer to similar elements and in which:
FIGS. 1A and 1B are exemplary conventional AI catheters.
FIG. 2A, 2B and 2C show deep rigid deep insemination catheters extending from conventional AI catheters.
FIGS. 3A and 3B are schematic views of the before and after deployment, respectively, of one embodiment of the catheter in accordance with the present invention.
FIGS. 4A through 4F show the assembly of the embodiment of the catheter of Figures 3A and 3B.
FIGS. 5A, 5B and 5C show one embodiment of the catheter attached to two exemplary AI dispensers.
FIGS. 5D and 5E show the catheter during and after deployment.

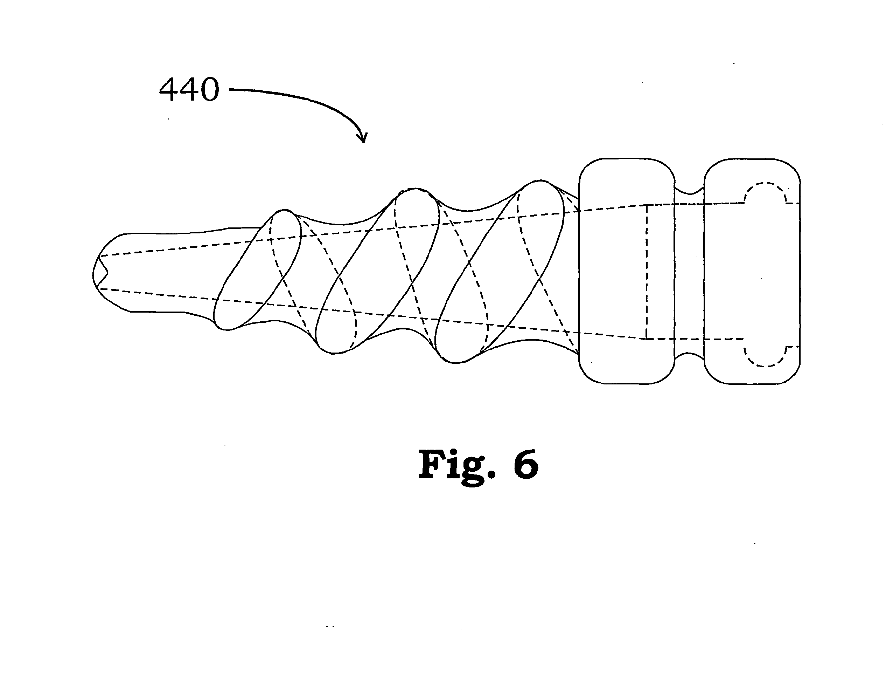

FIG. 6 is an enlarged drawing of one embodiment of a tapered nozzle for the catheter.
FIGS. 7A through 7E show the insertion and deployment of the catheter in a sow.
FIGS. 8A, 8B and 8C are cross-sectional views of alternative embodiments of the membrane for the catheter.
FIGS. 9A through 4D show the assembly and deployment of another embodiment of the catheter of Figures 3A and 3B.
FIGS. 10 and 11 show alternative embodiments of the catheter tube of Figure 9A.
FIGS. 12A through 12C show another embodiment of a catheter nozzle of the present invention.
FIGS. 12D and 12E show yet another embodiment of the catheter nozzle.
FIGS. 13A through 13D illustrate the controlled herniation during the deployment of the membrane in accordance with the invention.
FIGS 14A through 14E show another herniating embodiment of the membrane of the present invention.
FIGS 15A and 15B show yet another embodiment of the invention where the membrane has a closed tip.
FIGS. 16A and 16B show another embodiment of the invention wherein a container is coupled to a nozzle.

### DETAILED DESCRIPTION OF THE PREFERRED EMBODIMENTS

The present invention will now be described in detail with reference to a few preferred embodiments thereof as illustrated in the accompanying drawings. In the following description, numerous specific details are set forth in order to provide a thorough understanding of the present invention. It will be apparent, however, to one skilled in the art, that the present invention may be practiced without some or all of these specific details. In other instances, well known process steps and/or structures have not been described in detail in order to not unnecessarily obscure the present invention.

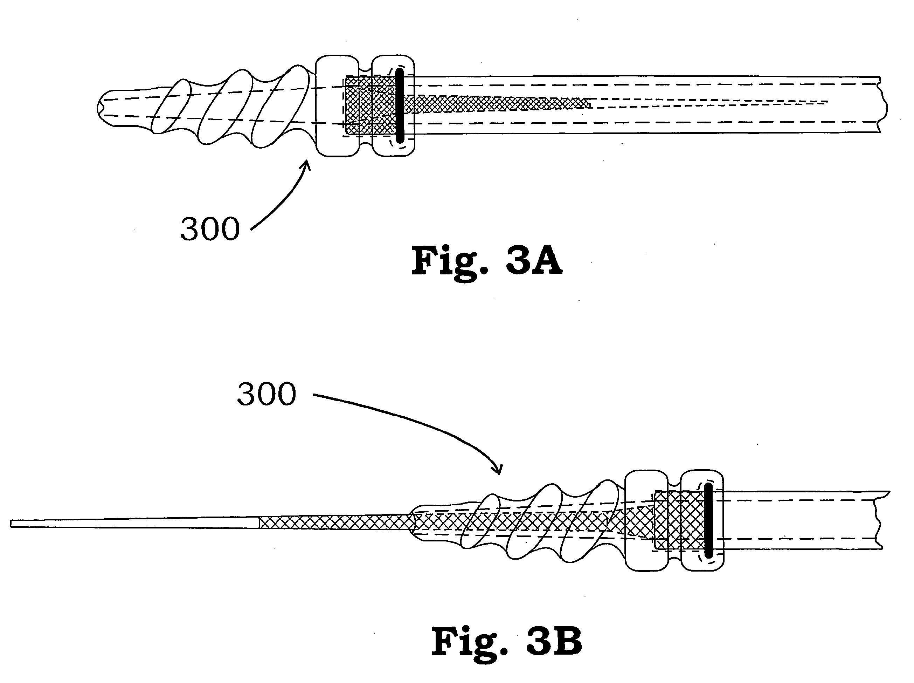

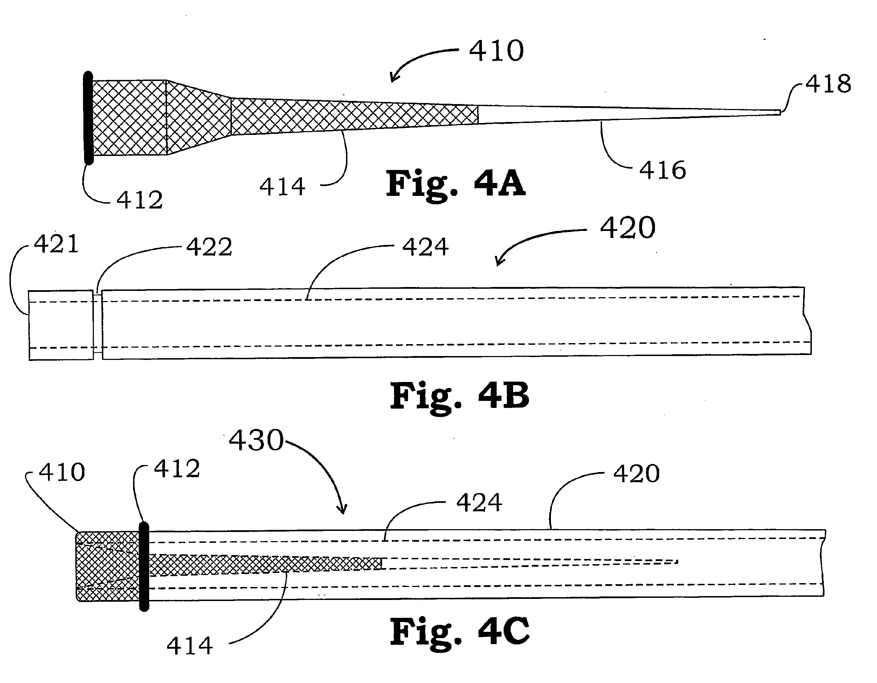

In accordance with the present invention, Figures 3A and 3B are views of one embodiment of catheter 300, prior to and after deployment of a membrane. Figures 4A through 4F illustrate the assembly of catheter 300 of Figures 3A and 3B.

Figures 4A, 4B and 4C show a membrane 410, a catheter tube 420, and a subassembly 430 comprising membrane 410 and tube 420. Membrane 410 can be attached to catheter tube 420 by inserting tip 418 of membrane 410 into opening 421 of tube 420, until deployable sections 414 and 416 of membrane of 410 are inside hollow 424 of tube 420. Next, a leading edge 412 of membrane 410 is snapped into a positioning ring 422 located on the outer surface of catheter tube 420, as shown in Figure 4C. Positioning ring 422 can be machined or molded depending on the manufacturing process. Other chemical and/or physical means of attaching membrane 410 to tube 420 can also be used, e.g., adhesive, heat bonding, ultrasonic welding, chemical bonding or heat staking.

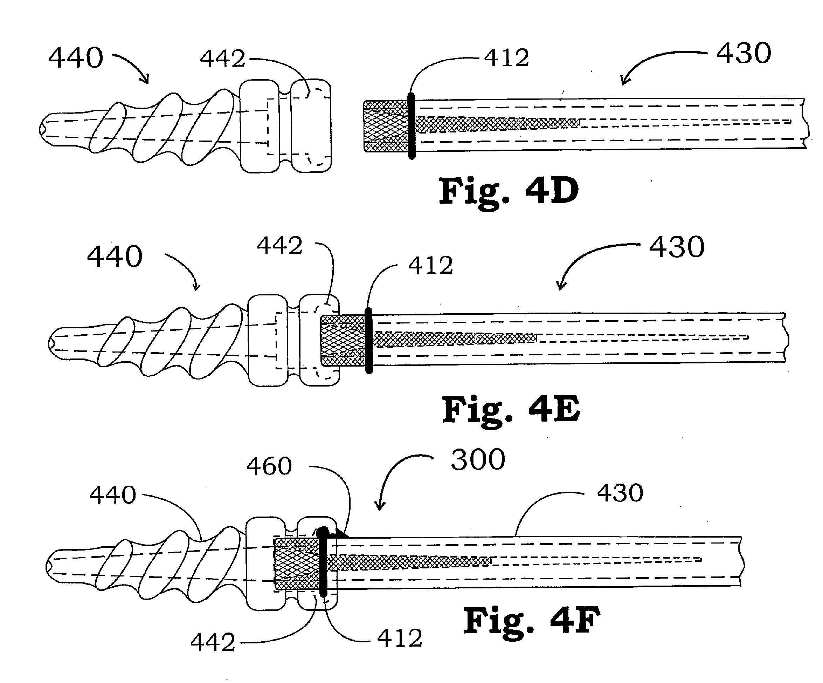

As shown in Figures 4D, 4E and 4F, subassembly 430 can be press fitted into catheter nozzle 440, by engaging membrane edge 412 of subassembly 430 into an internal positioning ring 442 of nozzle 440. Although subassembly 430 can be sufficiently mechanically coupled to nozzle 440, the various components of assembled catheter 300 can be further secured to each other by sonically welded or heat staked to prevent separation during deployment, such as inside the reproductive tract during artificial insemination (AI).

Alternatively, subassembly 430 can be replaced by a one-piece membrane-tube combination that can be manufactured by, for example, blow molding. Another method for constructing subassembly 430 is to insert catheter tube 420 over a membrane die, similar to dies used in balloon manufacturing, dipping the die and the attached catheter tube 420 into a suitable liquid membrane media until the entire die and about half inch of the end of catheter tube 420 is coated with the membrane media. After the liquid membrane media is cured, membrane tip 418 is cut. A downward movement of catheter tube 420 detaches tube 420 from the die and also automatically inverts membrane 410 into catheter tube 420, thereby forming subassembly 430.

Membrane tip 418 can include an opening such as a slit or a circular or oval hole. Alternatively, instead of an opening, tip 418 can include a soluble plug or a pre-weakened seal designed to dissolve or fail under pressure at the right time.

Depending on the specific application, nozzle 440 can be of different shapes and sizes, and combination thereof, including but not limited to spirals, bulbous knobs, including the nozzles illustrated by Figures 1A, 1B, 2A, 2B, and 2C. Although spirals are optional, approximately one to three spirals may be optimal when catheter 300 is used in swine. Shorter nozzles are also possible because membrane 410 is self-sealing, longer and self-guiding. In some embodiments, nozzle 440 is tapered to aid in insertion into the tract.

Different membrane materials and size thickness depend on applications and target animal. For virgin sows, also known as gilts, nozzle 440 may have a smaller diameter and shorter length. Conversely, for second to seventh parity sows with larger birth canals, nozzle 440 may have a larger diameter and longer length to facilitate the deposit of genetic materials and/or diagnostic instruments. For example in sows, the overall length of membrane 410 can be approximately 10,16 cm to 20,32 cm (four to eight inches) and tapering gently from 3,175 mm (one-eighth of an inch).

Depending on the specific type and size of the target application, different materials, size, and thickness can be employed. Suitable materials for nozzle 440 and membrane 410 of catheter 300 include silicone, silicone gel packs, foam, latex, ClearTex^{™} (a vailable from Zeller International, New York), polymers, plastics, metals, or combinations thereof. Other candidate materials include the polyolefms, polyethylene and polypropylene, the polyacetals, ploy-butadiene-styrene copolymers, the polyfluoro and polyfluorochloro-polymers, such as Teflon^{™} and other polymers and copolymers.

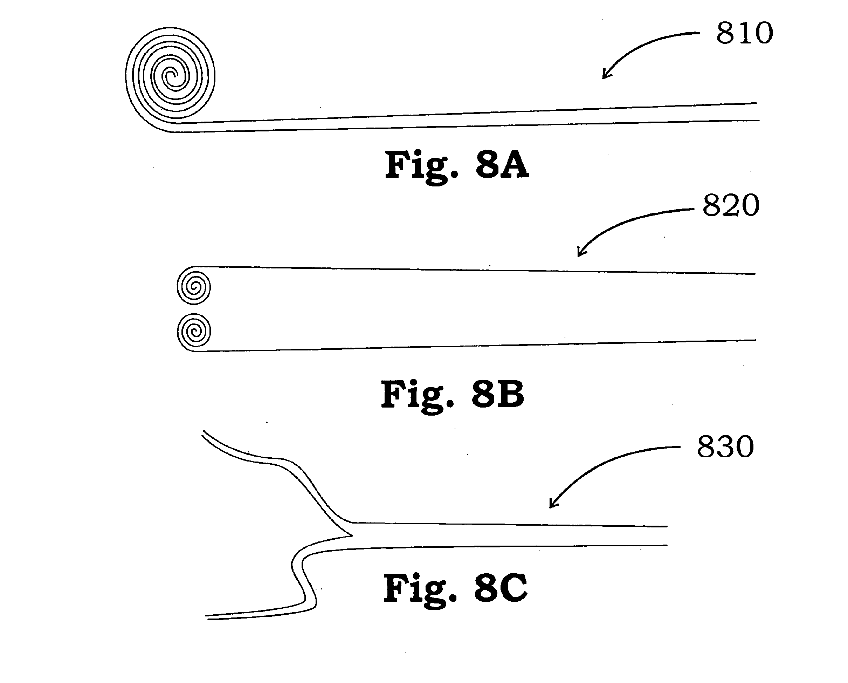

As shown in the cross-sectional views of Figures 8A and 8B, other embodiments include a membrane 810 that are similar to a children's party noisemaker and an inwardly-rolled embodiment 820 not unlike a condom, respectively. A twin forked-membrane 830 is also possible for deployment into the dual uterine horns of a sow, as shown in Figure 8C.

Many variations of catheter 300 are possible. For example, catheter 300 may have multiple tubes with multiple membranes. Such an embodiment may be useful in laparoscopy where one pathway is created for a camera and a second pathway is created for an instrument during surgery. Alternatively, a large diameter catheter 300 can also be used to create a large pathway within which one or more smaller catheters can be deployed.

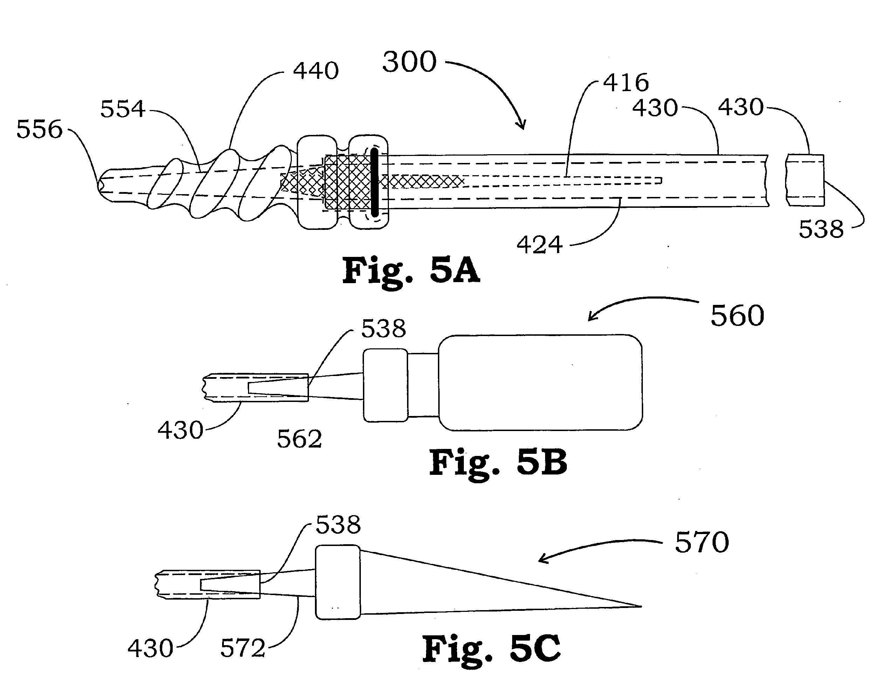

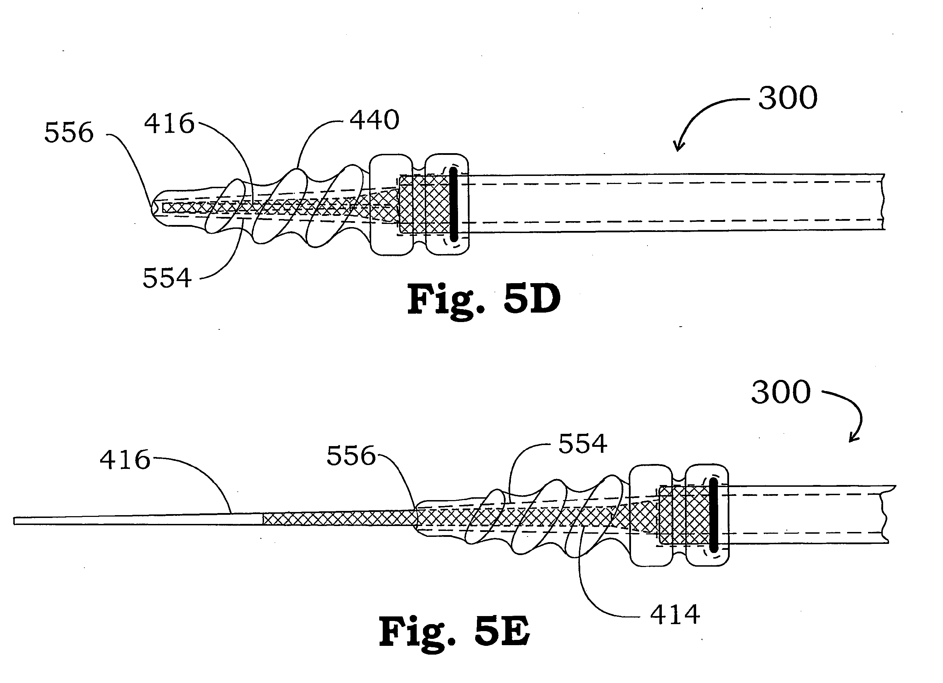

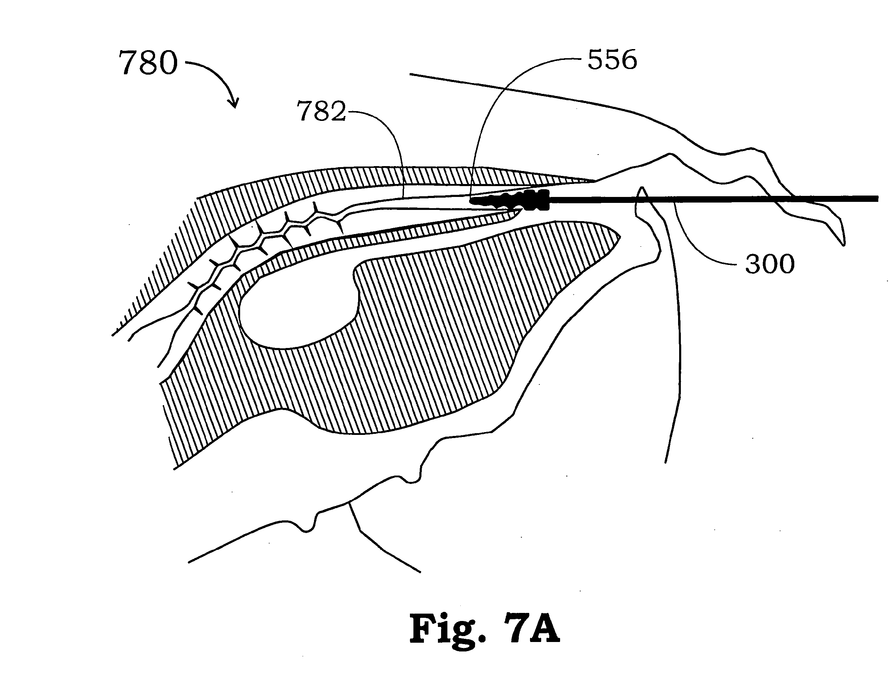

Figures 5A, 5D, and 5E, show catheter 300, before, during and after deployment, respectively. Figures 5B and 5C one embodiment of the catheter attached to two types of AI dispensers. Figures 7A through 7E show the insertion and deployment of catheter 300 in a sow 780. Catheter 300 is deployed by introducing genetic material suspended in a suitable fluid under pressure into sow 780. As shown in Figures 5B and 5C, the AI fluid can be transported in a suitable dispenser, such as a squeeze bottle 560 or a prepackaged tube 570.

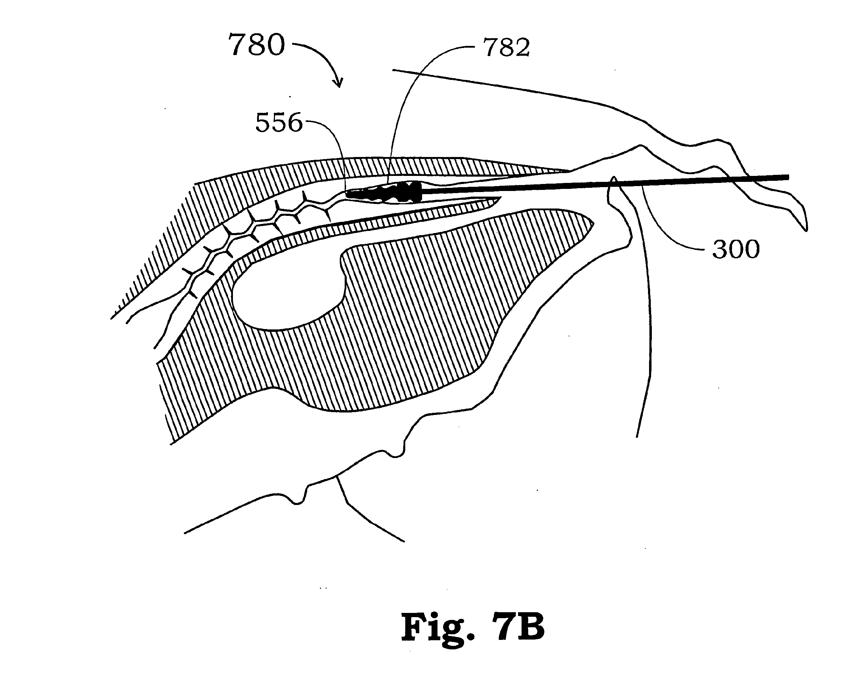

Referring to Figure 7A, catheter 300 is inserted into vaginal cavity 782 of sow 780. Catheter 300 is gradually pushed further into sow 780 until nozzle tip 556 is fully inserted into vagina cavity 782, as shown in Figure 7B.

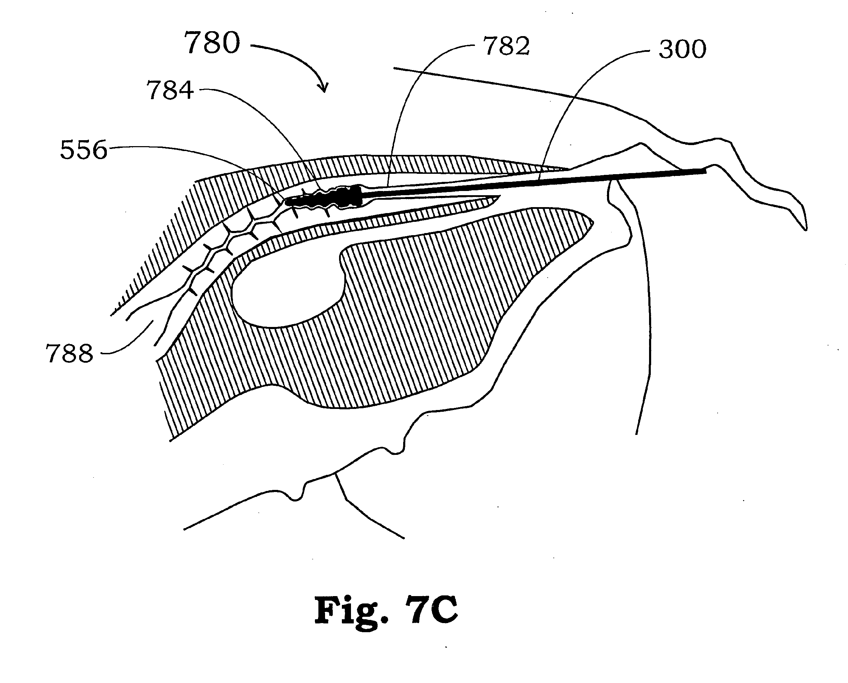

In Figure 7C, catheter 300 is then gently eased into cervical tract 784 of sow 780 until nozzle tip 556 engages at least the first cervical ring of cervical tract 784. Unlike conventional catheters, membrane 410 is not advanced until catheter 300 is positioned in cervical tract 784, thereby preventing contaminated materials that may be contained in vaginal cavity 782, or fluids from cervical tract 784, from being accidentally transferred into uterus 788 or uterine horns of sow 780. Hence, bio-security of uterus 788 is maintained.

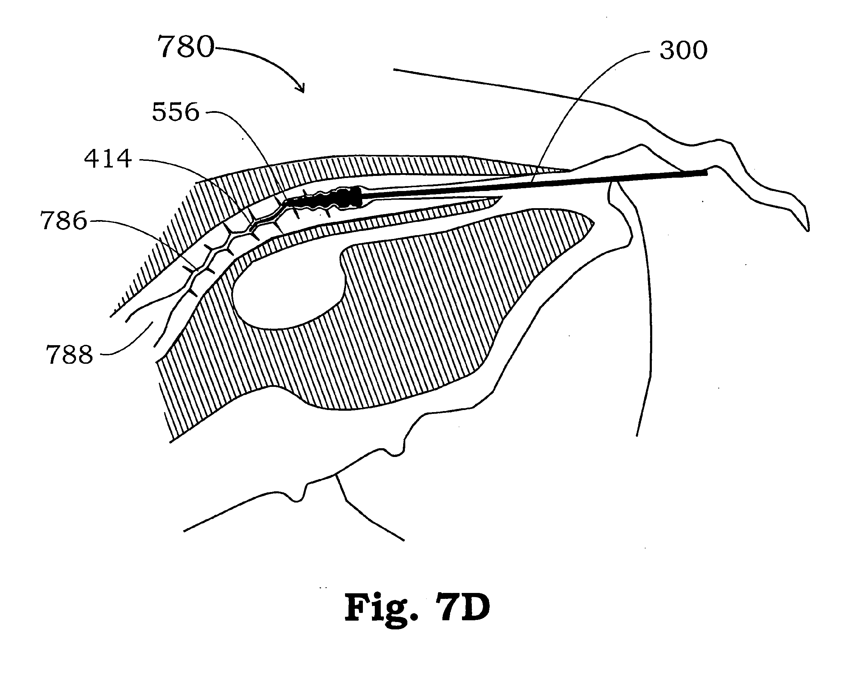

Next, as shown in Figure 7D, AI fluid under pressure is fed into catheter 300. Pressure can be generated manually via a dispenser 560 or by a suitable pump, such as a pneumatic or hydraulic pump. The effect of the pressure causes membrane 410 to begin unfolding in an inside-out manner not unlike removing one's sock by pulling from the open end. Although catheter 300 includes an opening in membrane tip 418, the AI fluid under pressure keeps the opening of tip 418 closed until membrane 410 is fully extended into cervical tract 784.

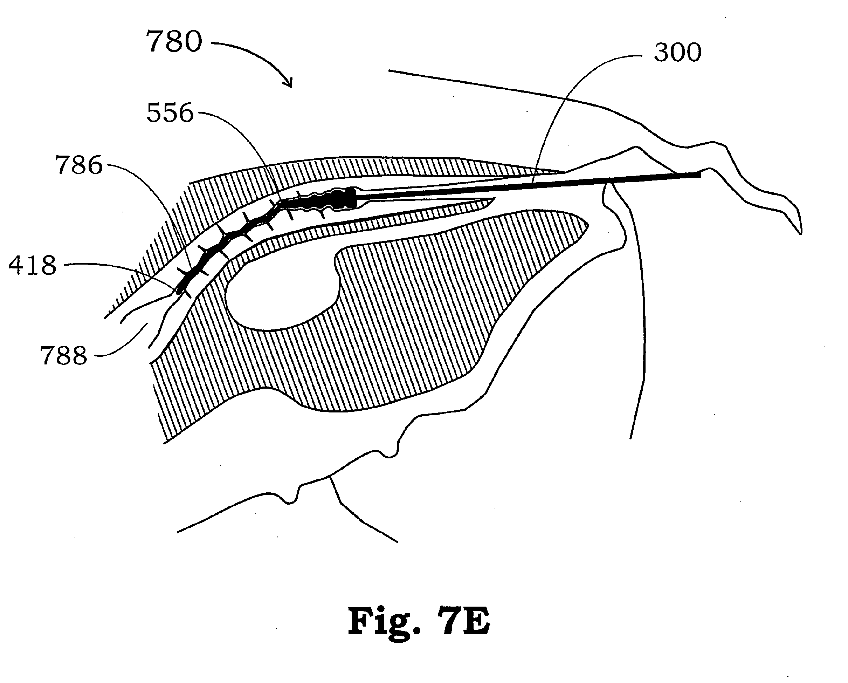

Referring now to Figure 7E, membrane 410 of catheter 300 continues to advance in a frictionless manner into the curved and narrow passageway of cervical tract 784, automatically centering the ever-expanding forward most portion of membrane 410 in the direction of least resistance. It is this expansion and automatic centering action of membrane 410 that advantageously enables membrane 410 to worm its way through cervical tract 784 without damaging or irritating delicate tissues. Eventually, when membrane 410 is fully extended and membrane tip 418 is near to or at the entrance of uterus 788, the pressure causes tip 418 to open thereby allowing the AI fluid to be deposited at the deeper end of cervical tract 786 and/or directly into uterus 788.

While a slight taper of membrane 410 aids deployment in cervical tract 786, the taper may not be necessary for proper deployment. In some applications, partial penetration of membrane 410 into the uterine horns (not shown) is also possible, allowing for example the introduction of embryo transplants.

Hence the invention eliminates the need for multiple removable sheaths by progressively feeding new portion of membrane 410 in an unfolding process. Every newly extended portion of membrane 410 is sterile because there is no prior contact with other biological tissue, such as vaginal cavity or other body fluids.

When a suitable amount of AI fluid has been deposited into sow 780, membrane 410 collapses after the fluid pressure dissipates, allowing for safe and easy withdrawal of the relatively flat, flexible, smooth and lubricated surface of membrane 410, causing minimal discomfort and posing minimal risk of trauma and damage to the recipient animal.

The use of trans-cervical intra-uterine AI advantageously reduces the volume of AI fluid needed for successful insemination by delivering the genetic materials where nature intended, i.e., into uterus 788. For example, a normal dose of 4-6 billion fresh swine semen may be reduced to fewer than 1 billion for successful AI when trans-cervical intra-uterine AI is employed.

In conventional AI, a small window of opportunity for a successful deposit of genetic material suspended in the AI fluid occurs during standing heat, which lasts for only five to eight minutes every one to three hours during estrus, when sow 780 is receptive to boar mounting. During standing heat, when a boar mounts sow 780, cervical tract 784 clamps onto the boar's penis to assist ejaculation, and uterine contractions draws the semen through cervical tract 784. If conventional AI is attempted outside this small window of opportunity, sow 780 will not assist in the drawing of the semen through cervical tract 784, and much of the AI fluid will backflow out the sow's vulva and is wasted, thereby reducing the probability of a successful litter.

Unlike conventional AI, catheter 300 is effective during refractory heat, which is the much longer period during estrus when cervical tract 784 is relaxed, allowing easier penetration of cervical tract 784. Since catheter 300 bridges cervical tract 784 and deposits the genetic material suspended in the AI fluid much closer to uterus 788, resistance caused by clamping cervical tract 784 during standing heat is not needed and probably undesirable. Hence catheter 300 is effective during the much longer refractory heat period because semen can be deposited efficiently and with minimal restriction in cervical tract 784.

Hence the advantages of trans-cervical intra-uterine AI can be combined with the relative safety and effectiveness of catheter 300 of the present invention. Farmers can now use AI in the much longer refractory heat period, allowing these swine farms to operate more efficiently, since successful AI is no longer limited to the much shorter standing heat period.

Yet another significant advantage of the present invention is the ability of membrane 410 to deploy in a self-centering and self-directing manner, when deployed under pressure. During manufacture, a suitable lubricant may be applied to the surface of membrane 410 that may come into contact with the tract of the animal, further reducing discomfort and risk of trauma during deployment and withdrawal of catheter 300.

In addition, unlike the conventional rigid deep penetration catheters, once membrane 410 of catheter 300 has been deployed and withdrawn from cervical tract 784, it is difficult to reinsert membrane 410 back into catheter nozzle 440 and tube 420, thereby discouraging the reuse of the now contaminated membrane 410.

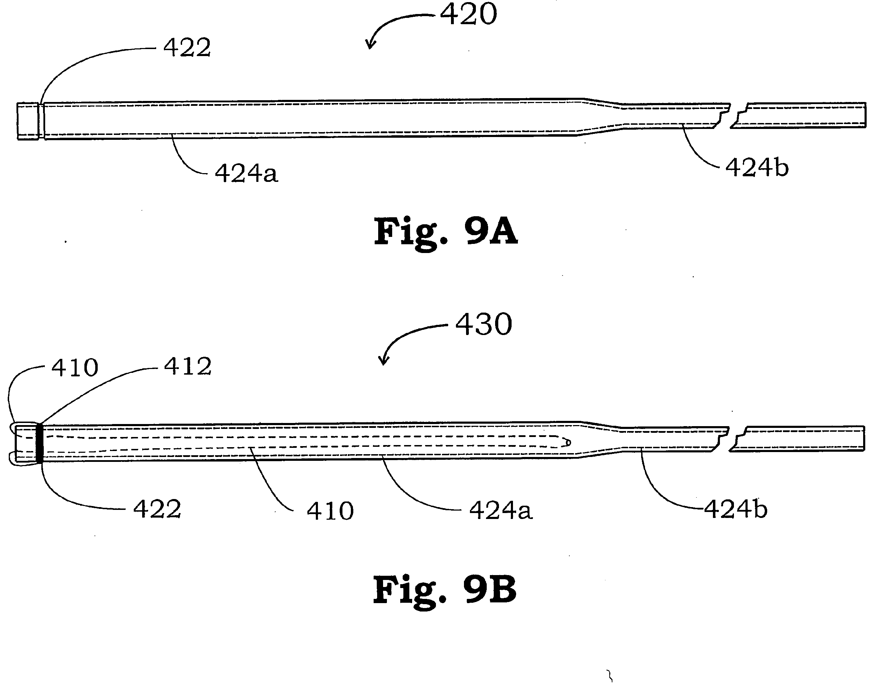

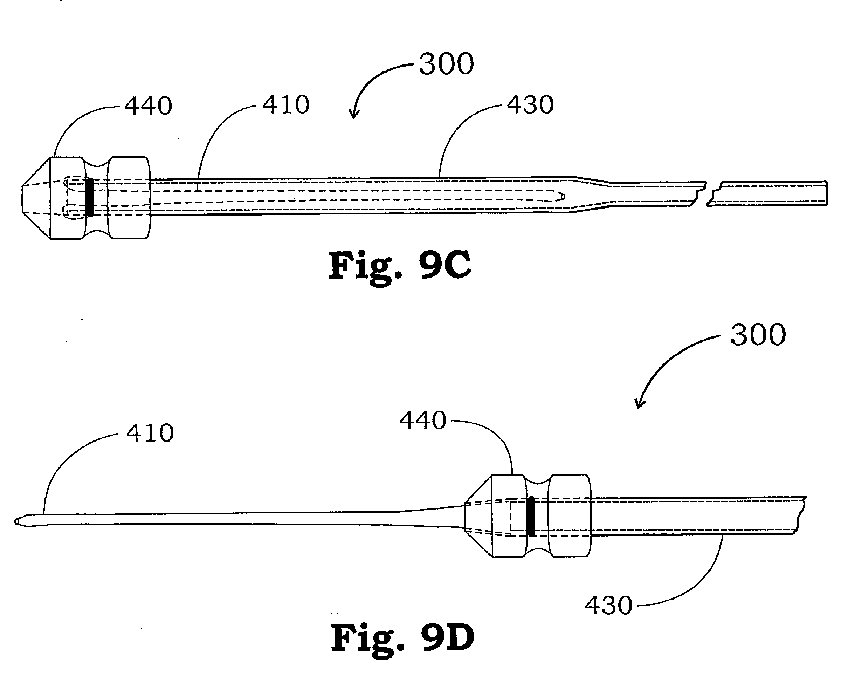

Referring now to Figures 9A through 9D, which show the assembly and deployment of another embodiment of the invention for use in fairly large animals such as swine, membrane 410 may have a fairly large diameter. This is because if the diameter is too small, membrane 410 may get trapped in the nooks and crannies between protrusions from the wall of cervical tract 784, 786. A fairly large diameter allows membrane 410 to gently push aside these protrusions thereby permitting membrane 410 to complete deployment.

However with a fairly large membrane diameter, any genetic material remaining inside catheter tube 420 after membrane 410 has fully deployed is wasted. Hence, in accordance with one aspect of the invention, tube 420 has a flared hollow section 424a and a reduced hollow section 424b. The flared aspect for tube 420 can be manufactured using techniques known to one skilled in the art including extrusion with vacuum or air pressure, blow-molding, and injection molding.

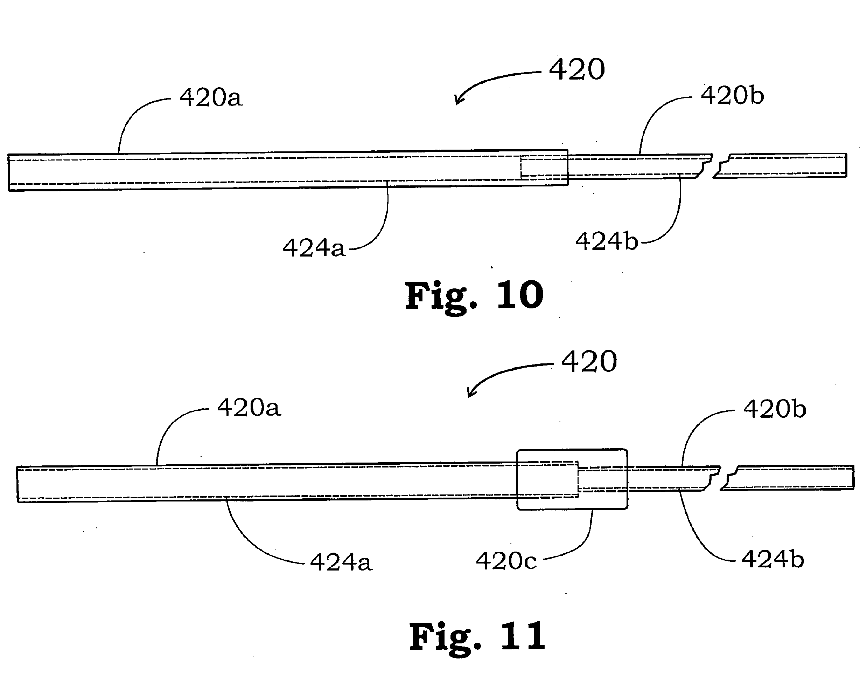

Figures 10 and 11 illustrate alternate embodiments of catheter tube 420 where a larger diameter tube section 420a is coupled to a smaller diameter tube section 420b. These sections 420a and 420b can be mated using techniques known to one skilled in the art such as press-fitting, adhesive or heat sealing, or with a connector 420c as shown in Figure 11.

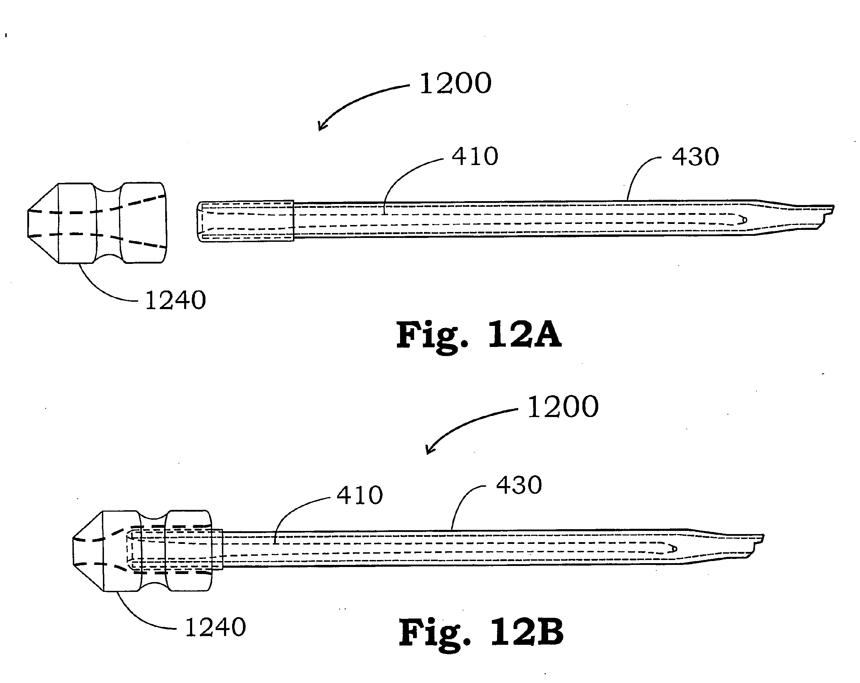

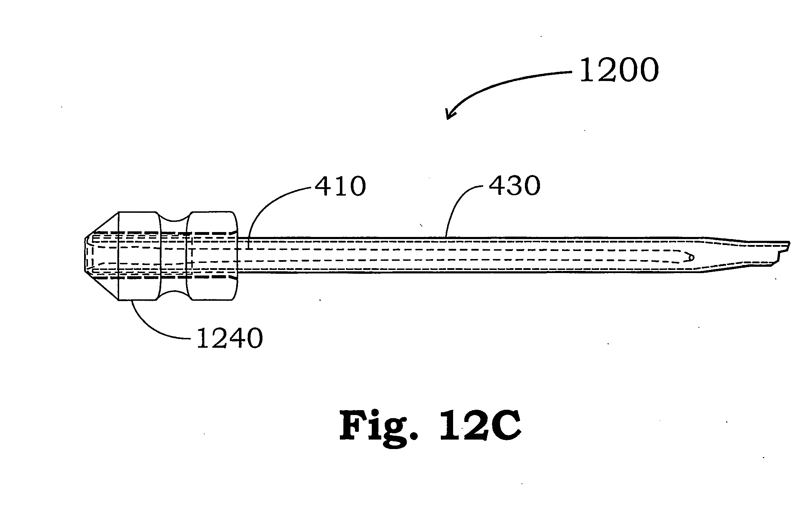

As discussed above, there are many ways to attach membrane 410 to catheter tube 420 forming subassembly 430, and also many ways to attach nozzle 440 to tube subassembly 430. Figures 12A-12C show the assembly of a self-sealing nozzle 1240 for catheter 1200, whereby the inner diameter of nozzle 1240 is tapered and is configured to fit securely thereby forming a tight seal with subassembly 430 when subassembly 430 is inserted into nozzle 1240.

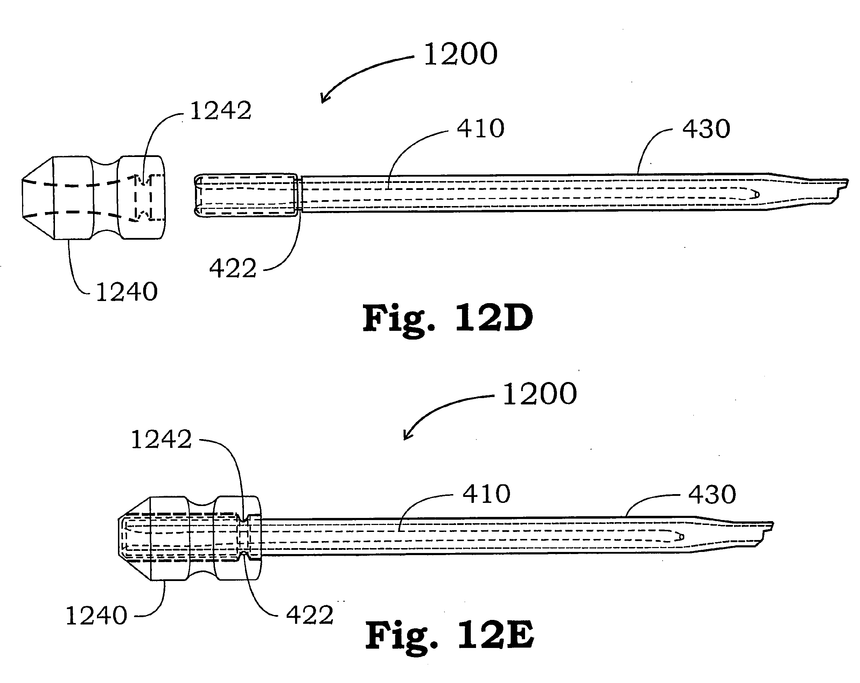

Figures 12D & 12E show the assembly of a snap-on version the self-Sealing nozzle 1240 of Figures 12A-12C. When subassembly 430 is inserted into nozzle 1240, an inner ring 1242 of nozzle 1240 snaps into a corresponding positioning ring 422 located on the outer surface of subassembly430.

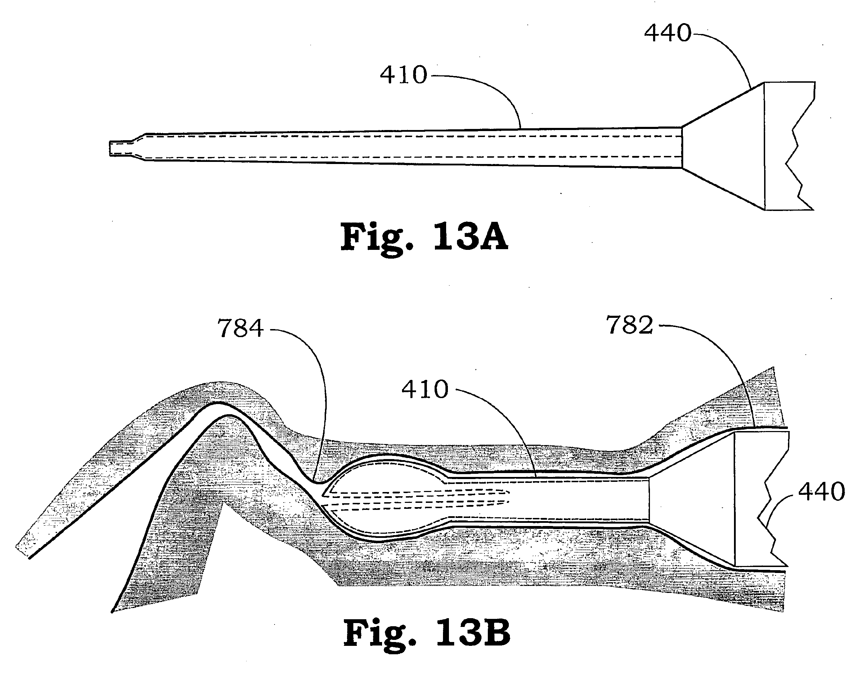

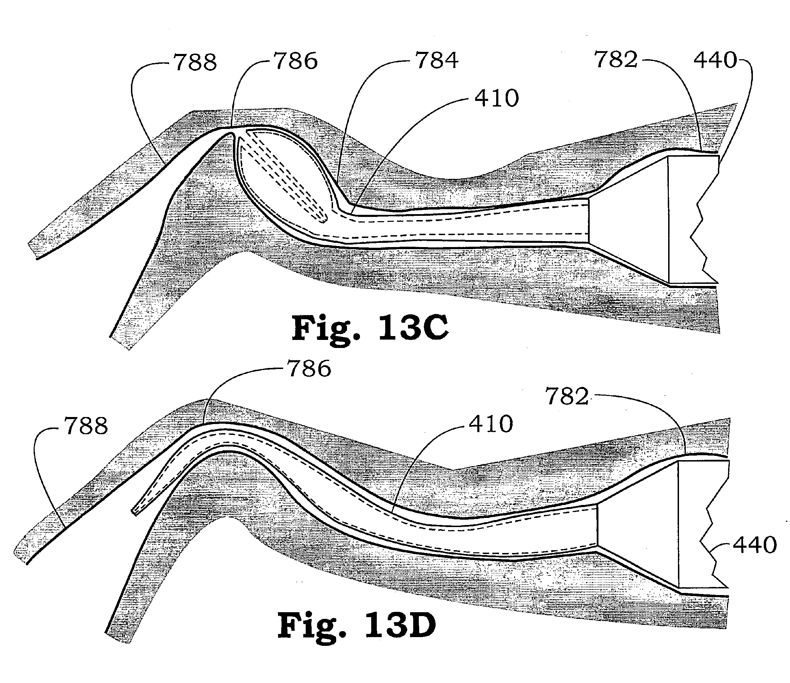

In accordance with another aspect of the invention, as shown by Figure 13A, deployment of membrane 410 through any obstructions such as a narrowing of cervical tract 784, 786 is facilitated by its tapered shape. Tapering of membrane 410 can be accomplished by reducing the wall thickness and/or diameter of membrane 410 towards its tip, i.e., away from nozzle 440. Hence, in some embodiments, the membrane wall thickness is tapered while the membrane diameter is gently tapered or not tapered at all.

For example in swine and similar size animals, the length of membrane 410 is approximately 7,62 cm to 17,78 cm (3 to 7 inches) from nozzle 440 to membrane tip. The external diameter of membrane 410 can range from about 0,48 cm to 0,79 cm (3/16 inches to 5/16 inches). Membrane wall thickness can vary from approximately 0,635 mm (0.025 inches) at nozzle 440 to 0,076 mm (0.003 inches) at the membrane tip.

Naturally, specific membrane dimensions will depend on the properties of membrane material such as elasticity and strength, and also depend on the size of the tract of the targeted animal species. Suitable membrane materials include a latex compound (product code 1175YL, batch X2471) available from Heveatex Corporation in Fall River, Massachusetts. Suitable coagulants include calcium nitrate tetra-hydrate crystal reagent, Ca(NO₃)₂.4H₂0.

Figures 13B through 13E illustrate the controlled herniation aspect of the present invention which is made possible by tapered membrane 410. In Figure 13B, membrane encounters an obstruction in the cervical tract 784. Because membrane 410 has a tapered wall thickness, herniation occurs at the point of least resistance, which is at the furthermost point of deployment in the cervical tract 784, i.e., the furthest point from nozzle 440. As shown in Figure 13c, with continuing fluid pressure in membrane 410, the herniation clears the obstruction at tract 784, enabling membrane 410 to deploy toward cervical tract 786. In this example, membrane 410 encounters another obstruction at cervical tract 786 and herniates at the present furthermost point of deployment, cervical tract 786, thereby clearing the obstruction and enabling membrane 410 to complete deployment into uterus 788.

There are several ways to manufacture membrane 410 with a tapered wall thickness. One way is to dip a membrane tool with the nozzle end first into a tank of liquid latex (not shown). By controlling the dwell time and dipping cycle in the tank, membrane 410 with a graduated wall thickness can be formed over the membrane tool. The combination of gravity and because the nozzle end of the membrane tool is the first to enter the tank and is also the last portion to leave the tank ensures that the wall thickness of membrane 410 is thickest near the nozzle end.

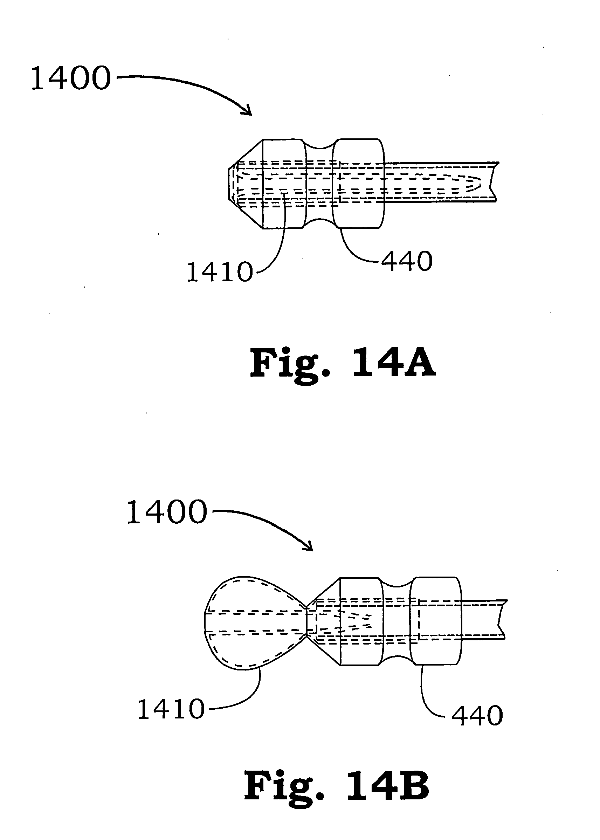

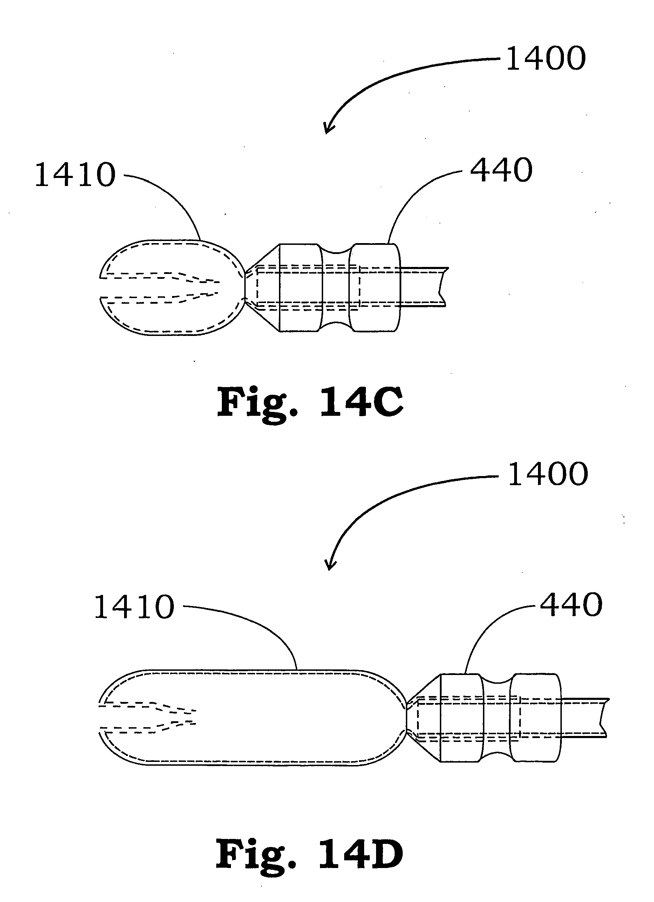

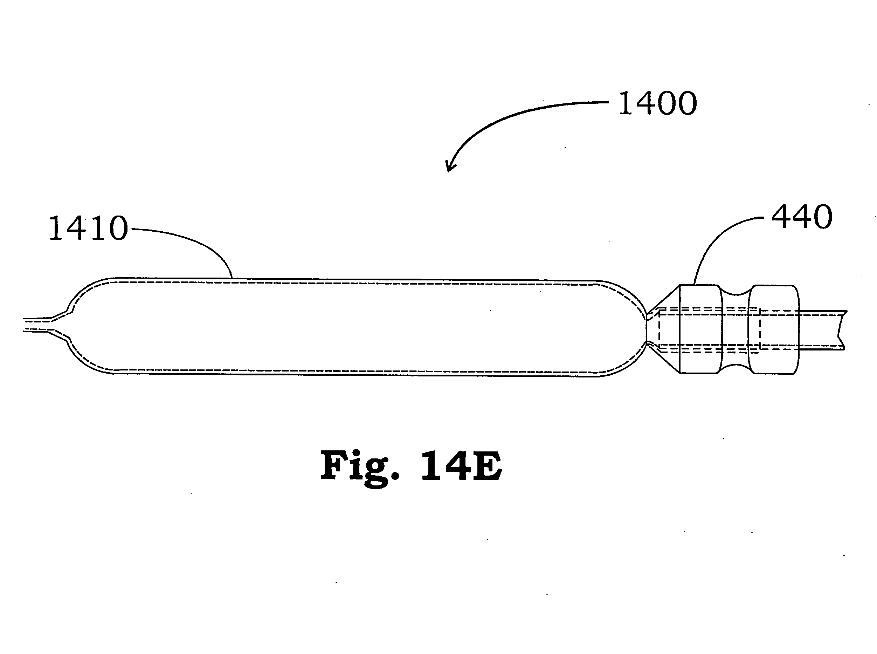

In another embodiment as shown in Figures 14A through 14E, instead of controlled herniation at the furthermost point of deployment, membrane 1410 herniates substantially continuously and uniformly along the tract during deployment. This embodiment may be useful in animals with relatively complex reproductive tracts, such as ewes, where the cervical tract comprises many potential traps for membrane 1410. By herniating and expanding continually to a controlled diameter, membrane 1410 fills the cervical tract and these traps can be gently pressed aside and rendering a relatively smooth pathway for the incoming genetic material.

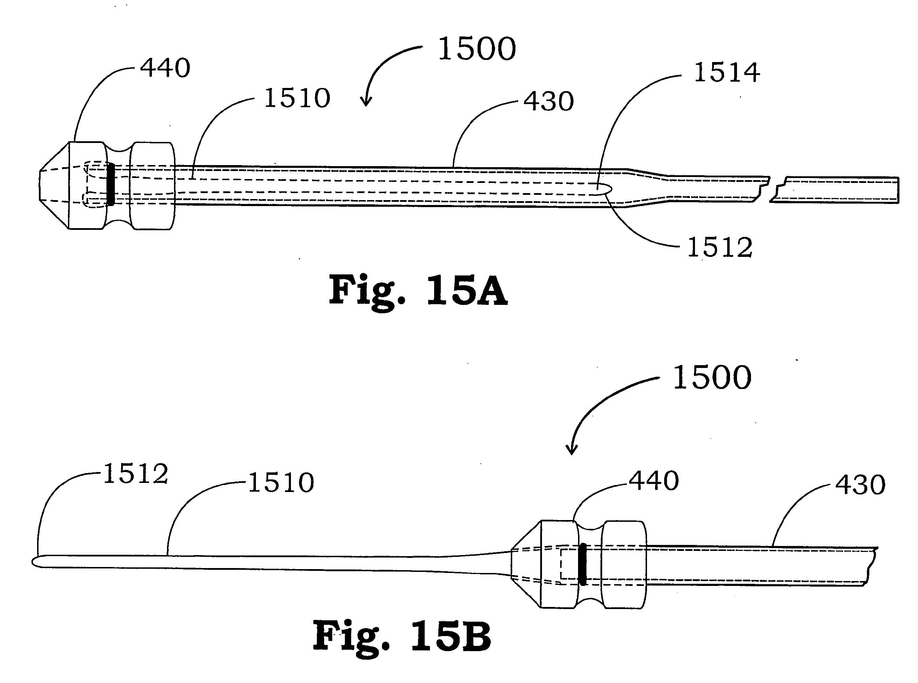

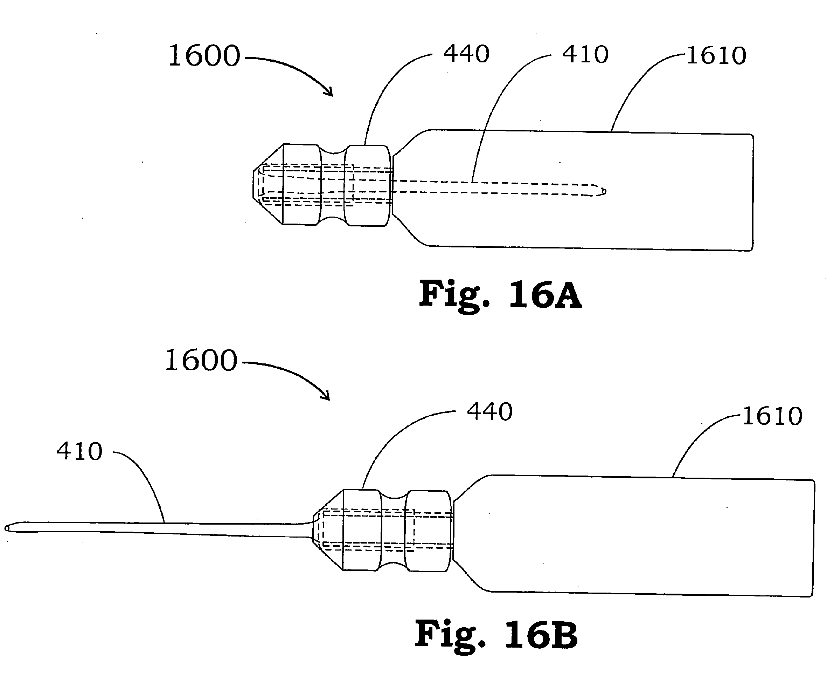

Figure 15A and 15B show another embodiment of the invention where membrane 1510 has a closed tip 1512. This closed tip 1512 enables the substantially precise placement of embryo(s) suspended in a minimal amount of fluid, or gel deposited at membrane pouch 1514. This embodiment may also be useful for depositing a small volume of genetic material such as previously frozen semen. Referring now to Figures 16A and 16B, in larger animal such as cows, a container 1610, e.g. a bottle or flexible semen tube (570), which holds the genetic material, can be inserted into the vagina of the animal. Hence, nozzle 440, attached to membrane 410, can be screwed directly onto container 1610 and the completed assembly 1600 is ready for deployment.

Once fully extended into a tract of a recipient animal, e.g., into the reproductive tract, respiratory tract, circulatory tract or digestive tract, catheter 300 provides a protective shield for the insertion of devices such as endoscopes, tracheal tubes, or other diagnostic and therapeutic instruments. Membrane 410 (the original patent references #416, unless I am misinterpreting...) shields the tract from the scraping, scarring and discomfort caused by the contact and friction of the hard, semi-blunt instruments and probes on the otherwise unprotected tract. As a result, healing time and the risk of infection are significantly reduced, thereby lowering recovery time and cost.

Although the described embodiment of catheter 300 uses an inverted membrane 410 which is turned inside-out during deployment, the concepts of a self-guiding, frictionless, membrane 410 which is deployed with minimal discomfort and trauma to recipient animals has many applications. In addition to AI and embryo transplant, many other applications for catheter 300 are possible. For example, catheter 300 can also be used for diagnostic and/or therapeutic applications in which pathways are created in the reproductive tract, respiratory tract, circulatory tract or digestive tract of the recipient animal or a patient. These pathways enable procedures such as embryo transplant and drug delivery to be performed. Laparoscopic procedures such as introducing cameras and instruments are also possible. Depending on the application, the size and shape of catheter 300 may vary.

While this invention has been described in terms of several preferred embodiments, there are alterations, modifications, permutations, and substitute equivalents, which fall within the scope of this invention. It should also be noted that there are many alternative ways of implementing the methods and apparatuses of the present invention.

## Claims

1. A catheter (300, 1200) for opening a pathway in the cervical tract of a sow and depositing an artificial insemination fluid into the sow's body, the catheter (300, 1200) comprising:
a tube (420) configured to be inserted into the cervical tract of the sow; and
a membrane (410, 830) coupled to said tube (420) and initially positioned inside said tube (420), wherein said membrane (410, 830) is configured to be extended from an opening (421) in said tube (420) and into the cervical tract by a pressure in the fluid to be deposited into the sow's body, said membrane (410, 830) extends without sliding action between the membrane (410) and the cervical tract, said membrane wall thickness is tapered off towards the distal tip and wherein the thinner membrane wall towards the distal tip (418) allows the membrane (410, 830) to herniate when the membrane (410, 830) encounters an obstruction in the cervical tract, thereby clearing the obstruction and enabling the membrane (410, 830) to continue to extend;
wherein the membrane (410) has an open tip, and wherein the opening of the tip can be kept closed by the fluid under pressure to be deposited into the sow's body until the membrane (410, 830) is fully extended into the cervical tract.

2. The catheter of claim 1, wherein the tube (420) has a tapered nozzle (440) located at the opening (421) of the tube (420).

3. The catheter of claim 2, wherein the nozzle (440) has a positioning ring (442) configured to mate with a corresponding positioning ring (422) on the tube (420).

4. The catheter of claim 2 or 3, wherein the nozzle (440) has a bigger outer diameter than the tube (420).

5. The catheter of any one of claims 1-4, wherein the membrane (410, 830) is tapered.

6. The catheter of any of claims 1-5, wherein the membrane (410, 830) is thin and pliable, thereby allowing the membrane to collapse after said pressure in the fluid dissipates.

7. The catheter of any of claims 1-6, wherein the membrane (410, 830) is twin forked.

8. The catheter of any of claims 1-7, wherein the tube (420) has a flared hollow section (424a) and a reduced hollow section (424b).

9. The catheter of any of claims 1-8, wherein the tube (420) has a larger diameter tube section (420a) coupled to a smaller diameter tube section (420b).

## Patentansprüche

1. Katheter (300, 1200) zum Öffnen eines Weges im Gebärmutterhals-Trakt einer Sau und zum Absetzen einer Flüssigkeit zur künstlichen Befruchtung in den Körper der Sau, wobei der Katheter (300, 1200) enthält:
ein Rohr (420), das dazu ausgelegt ist, in den Gebärmutterhals-Trakt der Sau eingeführt zu werden,
eine mit dem Rohr (420) verbundene und anfangs innerhalb des Rohres (420) positionierte Membran (410, 830), wobei die Membran (410, 830) dazu ausgelegt ist, durch einen Druck in der in den Körper der Sau abzusetzenden Flüssigkeit aus einer Öffnung (421) im Rohr (420) und in den Gebärmutterhals-Trakt ausgefahren zu werden, wobei die Membran (410, 830) ohne eine Gleitbewegung zwischen der Membran (410) und dem Gebärmutterhals-Trakt ausfährt, wobei die Wanddicke der Membran zur distalen Spitze hin abnimmt, und wobei es die dünnere Membranwand zur distalen Spitze (418) hin der Membran (410, 830) erlaubt, einen Druck auszuüben, wenn die Membran (410, 830) auf ein Hindernis im Gebärmutterhals-Trakt trifft, wodurch das Hindernis beseitigt wird und die Membran (410, 830) in die Lage versetzt wird, weiter auszufahren,
wobei die Membran (410) eine offene Spitze hat, und wobei die Öffnung der Spitze durch die unter Druck stehende Flüssigkeit, die in den Körper der Sau abzusetzen ist, geschlossen gehalten werden kann bis die Membran (410, 830) voll in den Gebärmutterhals-Trakt ausgefahren ist.

2. Katheter nach Anspruch 1, wobei das Rohr (420) eine sich verjüngende Düse (440) hat, die an der Öffnung (421) des Rohres (420) vorgesehen ist.

3. Katheter nach Anspruch 2, wobei die Düse (440) einen Positionierungsring (442) hat, der dazu ausgelegt ist, mit einem zugeordneten Positionierungsring (422) am Rohr (420) in Eingriff zu kommen.

4. Katheter nach Anspruch 2 oder 3, wobei die Düse (440) einen größeren Außendurchmesser als das Rohr (420) hat.

5. Katheter nach einem der Ansprüche 1-4, wobei sich die Membran (410, 830) verjüngt.

6. Katheter nach einem der Ansprüche 1-5, wobei die Membran (410, 830) dünn und biegsam ist, wodurch die Membran kollabieren kann, nachdem der Druck in der Flüssigkeit verschwindet.

7. Katheter nach einem der Ansprüche 1-6, wobei die Membran (410, 830) zwillingsgabelförmig ist.

8. Katheter nach einem der Ansprüche 1-7, wobei das Rohr (420) einen aufgeweiteten Hohlraumabschnitt (424a) und einen verkleinerten Hohlraumabschnitt (424b) hat.

9. Katheter nach einem der Ansprüche 1-8, wobei das Rohr (420) einen Rohrabschnitt (420a) mit größerem Durchmesser hat, der mit einem Rohrabschnitt (420b) mit kleinerem Durchmesser verbunden ist.

## Revendications

1. Cathéter (300, 120C) pour ouvrir une voie dans le tractus cervical d'une truie, et pour déposer un fluide d'insémination artificielle dans le corps de la truie, le cathéter (300, 1200) comprenant:
un tube (420) qui est configuré de manière à être inséré dans le tractus cervical de la truie; et
une membrane (410, 830) qui est couplée audit tube (420) et qui est initialement positionnée à l'intérieur dudit tube (420), dans lequel ladite membrane (410, 830) est configurée de manière à être étendue à partir d'une ouverture (421) dans ledit tube (420) et dans le tractus cervical par une pression dans le fluide à déposer dans le corps de la truie, ladite membrane (410, 830) s'étend sans action de glissement entre la membrane (410) et le tractus cervical, l'épaisseur de la paroi de ladite membrane se réduit en direction de la pointe distale, et dans lequel la paroi plus mince de la membrane en direction de la pointe distale (418) permet à la membrane (410, 830) de faire hernie lorsque la membrane (410, 830) rencontre une obstruction dans le tractus cervical, éliminant ainsi l'obstruction et permettant à la membrane (410, 830) de continuer à s'étendre;
dans lequel la membrane (410) présente une pointe ouverte, et dans lequel l'ouverture de la pointe peut être maintenue fermée par le fluide sous pression à déposer dans le corps de la truie jusqu'à ce que la membrane (410, 830) soit complètement étendue dans le tractus cervical.

2. Cathéter selon la revendication 1, dans lequel le tube (420) comprend une buse conique (440) qui est disposée à l'ouverture (421) du tube (420).

3. Cathéter selon la revendication 2, dans lequel la buse (440) comprend un anneau de positionnement (442) qui est configuré de manière à coïncider avec un anneau de positionnement correspondant (422) prévu sur le tube (420).

4. Cathéter selon la revendication 2 ou 3, dans lequel la buse (440) présente un diamètre extérieur supérieur à celui du tube (420).

5. Cathéter selon l'une quelconque des revendications 1 à 4, dans lequel la membrane (410, 830) est conique.

6. Cathéter selon l'une quelconque des revendications 1 à 5, dans lequel la membrane (410, 830) est mince et pliable, permettant de ce fait à la membrane de s'écraser une fois que ladite pression dans le fluide s'est dissipée.

7. Cathéter selon l'une quelconque des revendications 1 à 6, dans lequel la membrane (410, 830) a la forme d'une fourche à deux branches.

8. Cathéter selon l'une quelconque des revendications 1 à 7, dans lequel le tube (420) présente une section creuse évasée (424a) et une section creuse réduite (424b).

9. Cathéter selon l'une quelconque des revendications 1 à 8, dans lequel le tube (420) comprend une section de tube de plus grand diamètre (420a) qui est couplée à une section de tube de plus petit diamètre (420b).
